# EUROPEAN PATENT APPLICATION

(11) **EP 4 265 714 A1**
(43) Date of publication of application: **25.10.2023**
(21) Application number: 22742760.6
(22) Date of filing: 12.01.2022
(51) Int. Cl.: C12N 5/071, A61K 47/46, A61K 9/06, A61K 9/70, A61L 27/52, A61L 27/36, A61L 27/38

(54) **DECELLULARIZED TISSUE-DERIVED EXTRACELLULAR MATRIX MODIFIED WITH PHENOL DERIVATIVE AND USE THEREOF**

(30) Priority: 20.01.2021 KR 20210008205
(71) Applicant: Cellartgen Inc., Seoul 03722 (KR)
(72) Inventor: CHO, Seung Woo, Seoul 03722 (KR); LEE, Jung Seung, Seoul 03722 (KR); CHOI, Yi Sun, Seoul 03722 (KR); JEON, Eun Je, Seoul 03722 (KR)
(74) Representative: Manitz Finsterwald Patent- und Rechtsanwaltspartnerschaft mbB
(86) International application number: PCT/KR2022/000510
(87) International publication number: WO 2022/158784

(57) **Abstract**

The present disclosure relates to a hydrogel including a decellularized tissue-derived extracellular matrix functionalized with a phenol derivative, and a composition for cell culture, a composition for promoting cell differentiation, a tissue adhesive composition, a composition for drug delivery, a composition for promoting tissue regeneration and a composition for tissue implantation, each including the hydrogel. The hydrogel can be usefully used in the field of tissue engineering.

## Description

### TECHNICAL FIELD

The present disclosure relates to a hydrogel including a decellularized tissue-derived extracellular matrix functionalized with a phenol derivative, uses thereof, and a method of preparing the same.

### BACKGROUND

Decellularization can minimize immune response by removing cells from tissue and mimicking a tissue-specific microenvironment with various extracellular matrices, polysaccharides and specific proteins and thus has attracted a lot of attention in the field of tissue engineering (Falguni Pati et al., Nature Communications, 5:3935, 2014; Yuehe Fu et al., J. Cell. Mol. Med., 20(4):740-749, 2016). In particular, a tissue-derived extracellular matrix obtained by decellularization can be made into a solution and then used for surface modification and hydrogel production. Thus, it can also be used as a biomaterial for cell culture, implantation and tissue regeneration.

However, in spite of their excellent tissue-specific functionality, conventional tissue-derived extracellular matrix-based hydrogels are crosslinked by self-assembly. Therefore, they have disadvantages such as requiring a long time to be formed and weak physical properties and thus have limitations in being grafted onto the latest medical engineering technologies such as long-term cell culture and 3D printing. Accordingly, there is a need to develop a tissue-derived extracellular matrix-based hydrogel which has enhanced physical properties and various tissue engineering applications while maintaining tissue-specific functionality.

### DISCLOSURE OF THE INVENTION

### PROBLEMS TO BE SOLVED BY THE INVENTION

The present disclosure is conceived to provide a hydrogel including a decellularized tissue-derived extracellular matrix functionalized with a phenol derivative.

The present disclosure is also conceived to provide a composition for cell culture including the hydrogel.

The present disclosure is also conceived to provide a composition for promoting cell differentiation including the hydrogel.

The present disclosure is also conceived to provide a composition for drug delivery including the hydrogel.

The present disclosure is also conceived to provide a composition for promoting tissue regeneration including the hydrogel.

The present disclosure is also conceived to provide a composition for tissue implantation including the hydrogel.

The present disclosure is also conceived to provide a method of preparing a hydrogel based on a decellularized tissue-derived extracellular matrix functionalized with a phenol derivative, including a process of functionalizing a decellularized tissue-derived extracellular matrix with a phenol derivative.

### MEANS FOR SOLVING THE PROBLEMS

In order to solve the above-described problems, the present disclosure provides a hydrogel including a decellularized tissue-derived extracellular matrix functionalized with a phenol derivative.

Further, the present disclosure provides a composition for cell culture including the hydrogel.

Furthermore, the present disclosure provides a composition for promoting cell differentiation including the hydrogel.

Moreover, the present disclosure provides a composition for drug delivery including the hydrogel.

Also, the present disclosure provides a composition for promoting tissue regeneration including the hydrogel.

Further, the present disclosure provides a composition for tissue implantation including the hydrogel.

Furthermore, the present disclosure provides a method of preparing a hydrogel based on a decellularized tissue-derived extracellular matrix functionalized with a phenol derivative, including a process of functionalizing a decellularized tissue-derived extracellular matrix with a phenol derivative.

### EFFECTS OF THE INVENTION

A hydrogel according to the present disclosure has improved physical properties and structural stability and thus can form a stable three-dimensional structure for a long time without the use of an additional polymer scaffold. Also, the hydrogel has high biocompatibility and low cytotoxicity and thus can culture stem cells and promote differentiation of stem cells and tissue regeneration. Further, the hydrogel or hydrogel patch can encapsulate a drug therein and thus enables continuous drug delivery. Furthermore, the hydrogel can promote tissue regeneration and can be used for tissue implantation. Thus, it can be usefully used in the field of tissue engineering.

### BRIEF DESCRIPTION OF THE DRAWINGS

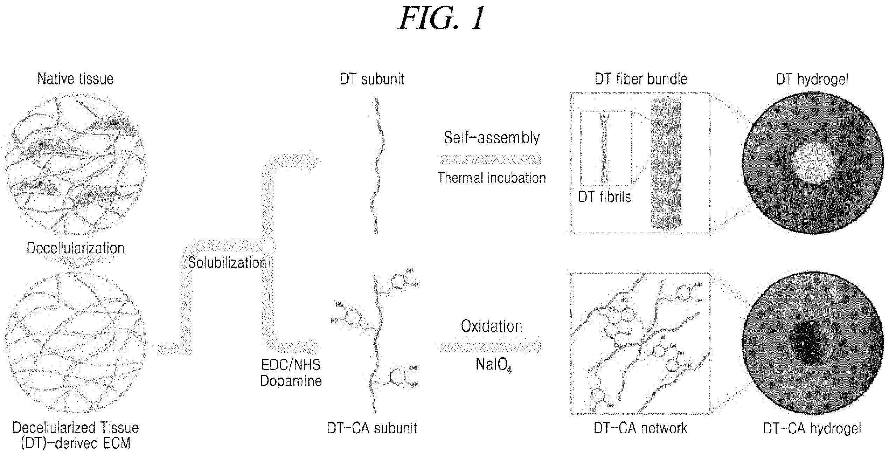

**FIG. 1** is a schematic diagram showing a process of preparing a decellularized tissue-derived extracellular matrix-based hydrogel functionalized with a catechol group (DT-CA hydrogel).
**FIG. 2A** shows a result of H&E staining and immunostaining for Col I in a tissue before decellularization (Before) and in the decellularized tissue (After).
**FIG. 2B** shows a result of analyzing the DNA content and the glycosaminoglycan (GAG) content in the tissue before decellularization (Before) and in the decellularized tissue (After).
**FIG. 2C** shows a result of ¹H-NMR of a decellularized tissue-derived extracellular matrix (DT) and a decellularized tissue-derived extracellular matrix functionalized with a catechol group (DT-CA) ((1) ethyl proton; and (2) phenyl proton).
**FIG. 3A** shows a result of observing an internal structure of each hydrogel.
**FIG. 3B** shows a result of measuring a relaxation stress of each hydrogel.
**FIG. 3C** shows a result of measuring a storage modulus (G') and a loss modulus (G") of each hydrogel.
**FIG. 3D** shows a result of measuring an elastic modulus of each hydrogel.
**FIG. 3E** shows a result of measuring the degree of elasticity of each hydrogel.
**FIG. 3F** shows a result of measuring a crosslinking reaction rate of each hydrogel (DT: DT hydrogel; tDT-CA: DT-CA hydrogel prepared by temperature crosslinking; and oDT-CA: DT-CA hydrogel prepared by oxidative crosslinking).
**FIG. 4A** shows a result of observing whether the shape of each hydrogel is maintained inside an aqueous solution (in solution) and outside the aqueous solution (in air).
**FIG. 4B** is a result of observing whether the shape of each hydrogel is maintained during additive manufacturing of each hydrogel (DT: DT hydrogel; and DT-CA: DT-CA hydrogel).
**FIG. 5A** shows a result of measuring a swelling ratio of each hydrogel in a PBS solution.
**FIG. 5B** shows a result of measuring a degradation ratio of each hydrogel in a collagenase solution.
**FIG. 5C** shows results of measuring tissue adhesion of each hydrogel (DT: DT hydrogel; and DT-CA: DT-CA hydrogel).
**FIG. 5D** shows results of measuring tissue adhesion of each hydrogel (DT: DT hydrogel; and DT-CA: DT-CA hydrogel).
**FIG. 6A** shows a result of measuring a shrinking ratio of each hydrogel with encapsulated stem cells (DT: DT hydrogel; and DT-CA: DT-CA hydrogel.
**FIG. 6B** shows results of cell viability in each hydrogel with encapsulated stem cells (Col-CA: collagen type I extracellular matrix-based hydrogel functionalized with a catechol group; and DT-CA: DT-CA hydrogel).
**FIG. 6C** shows results of cell viability in each hydrogel with encapsulated stem cells (Col-CA: collagen type I extracellular matrix-based hydrogel functionalized with a catechol group; and DT-CA: DT-CA hydrogel).
**FIG. 7A** shows a result of measuring mRNA expression levels of osteogenic differentiation markers osteopontin (OPN) and osteocalcin (OCN) after inducing osteogenic differentiation in each hydrogel with encapsulated stem cells.
**FIG. 7B** shows a result of immunostaining for OPN and fibronectin (FN).
**FIG. 7C** shows a result of Alizarin red staining.
**FIG. 7D** is a schematic diagram showing a test procedure in a calvaria defect animal model.
**FIG. 7E** shows results of Goldner's Trichrome staining and immunostaining for OPN and Col I after each hydrogel with encapsulated stem cells is implanted into the calvaria defect animal model (DT: DT hydrogel; Col-CA: collagen type I extracellular matrix-based hydrogel functionalized with a catechol group; and DT-CA: DT-CA hydrogel).
**FIG. 7F** shows results of Goldner's Trichrome staining and immunostaining for OPN and Col I after each hydrogel with encapsulated stem cells is implanted into the calvaria defect animal model (DT: DT hydrogel; Col-CA: collagen type I extracellular matrix-based hydrogel functionalized with a catechol group; and DT-CA: DT-CA hydrogel).
**FIG. 8A** shows a bone tissue before decellularization (Before) and the decellularized bone tissue (After)
**FIG. 8B** shows a decellularized bone tissue-derived extracellular matrix-based hydrogel (Bone hydrogel) prepared by temperature crosslinking and a decellularized bone tissue-derived extracellular matrix-based hydrogel functionalized with a catechol group (Bone-CA hydrogel) prepared by oxidative crosslinking.
**FIG. 8C** shows a result of measuring a storage modulus (G') and a loss modulus (G") of each hydrogel.
**FIG. 8D** shows a result of measuring an elastic modulus of each hydrogel.
**FIG. 8E** shows a result of measuring the degree of elasticity of each hydrogel.
**FIG. 9** shows a result of measuring the degree of release of VEGF over time after VEGF is encapsulated in each hydrogel (DT: DT hydrogel; and DT-CA: DT-CA hydrogel).
**FIG. 10A** shows DT-CA hydrogel patches prepared in various shapes.
**FIG. 10B** shows a result of measuring a storage modulus (G'), a loss modulus (G") and the degree of elasticity of each DT-CA hydrogel patch.
**FIG. 10C** shows a result of measuring the amount of a drug released over time after culturing each DT-CA hydrogel patch with an encapsulated drug (EGF) in a PBS or collagenase A solution.
**FIG. 11A** shows results of comparing the sizes of wounds after implanting hydrogels into wound animal models, respectively.
**FIG. 11B** shows results of comparing the sizes of wounds after implanting hydrogels into wound animal models, respectively.
**FIG. 11C** shows results of measuring a blood flow rate in a wound area of each mouse on day 12 of material implantation (NT: negative control; Bulk: solution-based DT-CA hydrogel; Patch: DT-CA hydrogel patch; Bulk+EGF: DT-CA hydrogel with encapsulated EGF (1 µg/mouse); and Patch+EGF: DT-CA hydrogel patch with encapsulated EGF (1 µg/mouse)).
**FIG. 11D** shows results of measuring a blood flow rate in a wound area of each mouse on day 12 of material implantation (NT: negative control; Bulk: solution-based DT-CA hydrogel; Patch: DT-CA hydrogel patch; Bulk+EGF: DT-CA hydrogel with encapsulated EGF (1 µg/mouse); and Patch+EGF: DT-CA hydrogel patch with encapsulated EGF (1 µg/mouse)).
**FIG. 12A** shows results of H&E staining, Masson's trichrome staining (MTS) and immunostaining for Keratin 14 and Involucrin in each wound area after implanting the hydrogels into the wound animal models, respectively.
**FIG. 12B** shows results of measuring the wound area, the epidermal thickness, the amount of collagen and the number of hair follicles in each wound area.
**FIG. 13A** is a schematic diagram showing a process of preparing a decellularized tissue-derived extracellular matrix-based hydrogel functionalized with a pyrogallol group (DT-PG hydrogel).
**FIG. 13B** shows a result of measuring a storage modulus (G'), a loss modulus (G") and an elastic modulus of each of 1% and 2% DT-PG hydrogels.
**FIG. 14A** shows results of comparing the sizes of wounds after implanting hydrogels into wound animal models, respectively.
**FIG. 14B** shows results of comparing the sizes of wounds after implanting hydrogels into wound animal models, respectively.
**FIG. 14C** shows results of measuring a blood flow rate in a wound area of each mouse on day 17 of material implantation (NT: negative control; Bulk: solution-based DT-PG hydrogel; Patch: DT-PG hydrogel patch; Bulk+EGF: DT-PG hydrogel with encapsulated EGF (1 µg/mouse); and Patch+EGF: DT-PG hydrogel patch with encapsulated EGF (1 µg/mouse)).
**FIG. 14D** shows results of measuring a blood flow rate in a wound area of each mouse on day 17 of material implantation (NT: negative control; Bulk: solution-based DT-PG hydrogel; Patch: DT-PG hydrogel patch; Bulk+EGF: DT-PG hydrogel with encapsulated EGF (1 µg/mouse); and Patch+EGF: DT-PG hydrogel patch with encapsulated EGF (1 µg/mouse)).
**FIG. 15A** shows results of H&E staining, Masson's trichrome staining (MTS) and immunostaining for Keratin 14 and Keratin 10 in each wound area after implanting the hydrogels into the wound animal models, respectively.
**FIG. 15B** shows results of measuring the wound area, the epidermal thickness, the amount of collagen and the number of hair follicles in each wound area.

### BEST MODE FOR CARRYING OUT THE INVENTION

The present disclosure relates to a hydrogel including a decellularized tissue-derived extracellular matrix functionalized with a phenol derivative.

In an example of the present disclosure, the phenol derivative may be a catechol group derived from a catechol-based compound selected from the group consisting of catechol, 4-tert-butylcatechol (TBC), urushiol, alizarin, dopamine, dopamine hydrochloride, 3,4-dihydroxyphenylalanine (DOPA), caffeic acid, norepinephrine, epinephrine, 3,4-dihydroxyphenylacetic acid (DOPAC), isoprenaline, isoproterenol and 3,4-dihydroxybenzoic acid; or a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol, but is not limited thereto.

In an example of the present disclosure, the tissue may be selected from the group consisting of liver, heart, kidney, muscle, stomach, intestine, lung, bone, cartilage, blood vessel, bladder, skin, brain, fat, thyroid gland, salivary gland, esophagus, pancreas, spinal cord, ligament, tendon, tooth and uterus, but is not limited thereto.

In an example of the present disclosure, 90% or more of cells may be removed from the decellularized tissue-derived extracellular matrix. Desirably, 95% or more of cells may be removed from the decellularized tissue-derived extracellular matrix. More desirably, 98% or more of cells may be removed from the decellularized tissue-derived extracellular matrix. More desirably, 99% or more of cells may be removed from the decellularized tissue-derived extracellular matrix.

In an example of the present disclosure, in the decellularized tissue-derived extracellular matrix functionalized with a phenol derivative, the hydrogel may be crosslinked through oxidation of the functionalized phenol derivative. The oxidation may be carried out by addition of an oxidizer or an enzyme. The oxidizer may be sodium periodate (NaIO₄) and hydrogen peroxide (H₂O₂), and the enzyme may be a peroxidase, but is not limited thereto.

Also, the oxidation may be carried out by a body enzyme without addition of an oxidizer or an enzyme. When the hydrogel is functionalized with a phenol derivative with stronger oxidative activity, for example, pyrogallol, it may be crosslinked through oxidation with a body enzyme without using an additional oxidizer or enzyme.

In an example of the present disclosure, the hydrogel may have a porous structure.

In an example of the present disclosure, the hydrogel may have an elastic modulus of from 100 Pa to 1500 Pa at 1 Hz. Desirably, the hydrogel may have an elastic modulus of from 200 Pa to 1500 Pa, from 300 Pa to 1500 Pa, from 400 Pa to 1500 Pa, from 500 Pa to 1500 Pa, from 600 Pa to 1500 Pa, from 700 Pa to 1500 Pa, from 800 Pa to 1500 Pa and from 900 Pa to 1500 Pa at 1 Hz, but is not limited thereto.

In an example of the present disclosure, the hydrogel may have a tissue adhesive property or may be biodegradable.

Further, the present disclosure provides a composition for cell culture including a hydrogel based on a decellularized tissue-derived extracellular matrix functionalized with a phenol derivative.

In an example of the present disclosure, the culture may be a three-dimensional culture. The cells may be stem cells, hematopoietic stem cells, hepatic cells, fibrous cells, epithelial cells, mesothelial cells, endothelial cells, muscle cells, nerve cells, immune cells, adipocytes, chondrocytes, bone cells, blood cells or skin cells, and can be applied to all cells capable of growth in the hydrogel of the present disclosure regardless of the cell type.

Furthermore, the present disclosure provides a composition for promoting cell differentiation including a hydrogel based on a decellularized tissue-derived extracellular matrix functionalized with a phenol derivative.

In an example of the present disclosure, the cell may be a stem cell, and the stem cell may be selected from the group consisting of embryonic stem cells, fetal stem cells, induced pluripotent stem cells and adult stem cells.

Moreover, the present disclosure provides a composition for drug delivery including a hydrogel based on a decellularized tissue-derived extracellular matrix functionalized with a phenol derivative.

In an example of the present disclosure, the drug may be encapsulated or loaded in the hydrogel, and may be selected from the group consisting of an immune cell activator, an anticancer agent, a therapeutic antibody, an antibiotic, an antibacterial agent, an antiviral agent, an anti-inflammatory agent, a contrast agent, a protein drug, a growth factor, a cytokine, a peptide drug, a hair growth agent, an anesthetic and combinations thereof, but is not limited thereto.

The anticancer agent may be selected from the group consisting of anthracyclines such as doxorubicin, daunomycin, epirubicin, idarubicin, etc.; taxanes such as paclitaxel, docetaxel, cabazitaxel, tesetaxel, etc.; alkaloids such as curcumin, camptothecin, berberine, evodiamine, matrine, piperine, sanguinarine, tetrandrine, thalicarpine, ellipticine, etc.; vinca alkaloids vinblastine, vincristine, vindesine, vinorelbine, etc.; platinums such as cisplatin, carboplatin, oxaliplatin, etc.; antimetabolites such as 5-fluorouracil, capecitabine, methotrexate, gemcitabine, etc.; topoisomerase inhibitors such as irinotecan, topotecan, etoposide, teniposide, etoposide, amsacrine, etc.; antitumor antibiotics such as bleomycin, actinomycin, mitomycin, mitoxantrone, etc.; alkylating agents such as cyclophosphamide, mechlorethamine, chlorambucil, melphalan, nitrosourea, etc.; nucleoside analogs such as azacytidine, azathioprine, cytarabine, doxifluridine, hydroxyurea, mercaptopurine, methotrexate, etc.; genetic drugs such as small interfering RNA (siRNA), small hairpin RNA (shRNA), microRNA (miRNA), plasmid DNA, etc.; enzymes such as L-asparaginase, etc.; hormones such as triptorelin acetate, megestrol acetate, flutamide, bicalutamide, goserelin, etc.; cytochrome C; and p53 protein, but is not limited thereto.

The therapeutic antibody may be selected from the group consisting of trastuzumab, rituximab, bevacizumab, cetuximab, bortezomib, erlotinib, gefitinib, imatinib mesylate, sunitinib, pembrolizumab, nivolumab, atezolizumab, ipilimumab and blinatumomab, but is not limited thereto.

The antibiotic may be selected from the group consisting of β-lactams, aminoglycosides, macrolides, tetracyclines, glycopeptides, lincosamides, quinolones, chloramphenicol, sulfa drugs, trimethoprim, polymyxin, bacitracin, mupirocin, fusidic acid, streptogramin and oxazolidinone, but is not limited thereto.

The antibacterial agent may be selected from the group consisting of metronidazole, secnidazole, ornidazole, tinidazole, clindamycin, sodium polystyrene sulfate and sodium cellulose sulfate, but is not limited thereto.

The antiviral agent may be selected from the group consisting of acyclobyl, vidarabine, ganciclovir, foscarnet, famciclovir, ribavirin, amantadine, zanamivir and oseltamivir, but is not limited thereto.

The anti-inflammatory agent may be selected from the group consisting of NSAIDs such as ibuprofen, dexibuprofen, naproxen, fenoprofen, ketoprofen, dexketoprofen, flurbiprofen, oxaprozin, loxoprofen, indomethacin, tolmetin, sulindac, etodolac, ketorolac, diclofenac, nabumetone, piroxicam, meloxicam, tenoxicam, droxicam, lornoxicam, isoxicam, mefenamic acid, meclofenamic acid, flufenamic acid, tolfenamic acid, niflumic acid, aspirin, diflunisal, salsalate and dexamethasone, but is not limited thereto.

The contrast agent may be selected from the group consisting of a PET contrast agent, a CT contrast agent, an MRI contrast agent, an optical image contrast agent and a US contrast agent, but is not limited thereto.

The growth factor may be selected from the group consisting of a vascular endothelial growth factor, an epidermal growth factor, a keratinocyte growth factor, a growth and differentiation factor, a hepatocyte growth factor, a platelet-derived growth factor, a transforming growth factor, angiopoietin, erythropoietin, a bone morphogenetic protein, an insulin-like growth factor, an acidic and basic fibroblast growth factor, a granulocyte-macrophage colony-stimulating factor, a brain-derived neurotrophic factor, a glial cell-derived neurotrophic factor, a nerve growth factor, a stromal cell-derived factor-1, a substance P and a hypoxia-inducible factor-1, but is not limited thereto.

The cytokine may be selected from the group consisting of interleukin, lymphokine, monokine, chemokine, interferon and adipokine, but is not limited thereto.

The hair growth agent may be selected from the group consisting of finasteride or dutasteride, but is not limited thereto.

The anesthetic may be selected from the group consisting of ropivacaine, bupivacaine, chloroprocaine, lidocaine, mepivacaine, procaine, tetracaine, levobupivacaine and articaine, but is not limited thereto.

In an example of the present disclosure, the composition may be in the form of an adhesive patch or a film.

Also, the present disclosure provides a composition for promoting tissue regeneration or for tissue implantation including a hydrogel based on a decellularized tissue-derived extracellular matrix functionalized with a phenol derivative.

In an example of the present disclosure, the tissue may be selected from the group consisting of liver, heart, kidney, muscle, stomach, intestine, lung, bone, cartilage, blood vessel, bladder, skin, brain, fat, thyroid gland, salivary gland, esophagus, pancreas, spinal cord, ligament, tendon, tooth and uterus, but is not limited thereto. Furthermore, the composition may be in the form of an adhesive patch or a film, and the drug may be encapsulated or loaded in the hydrogel.

Furthermore, the present disclosure provides a method of preparing a hydrogel based on a decellularized tissue-derived extracellular matrix functionalized with a phenol derivative, including a process of functionalizing a decellularized tissue-derived extracellular matrix with a phenol derivative and a process of oxidizing the functionalized phenol derivative. The oxidation may be carried out by addition of an oxidizer or an enzyme, or may be carried out by a body enzyme without addition of an oxidizer or an enzyme.

The pharmaceutical composition according to the present disclosure may be prepared into a pharmaceutical dosage form by a well-known method in the art, so that an active component of the composition may be provided via a fast, suspended or prolonged release, after being administered into a mammal. When preparing a dosage form, the pharmaceutical composition according to the present disclosure may further contain a pharmaceutically acceptable carrier, to the extent that this carrier does not inhibit a function of the active component.

The pharmaceutically acceptable carrier may include commonly-used carriers, such as pectin, hyaluronic acid, collagen, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil, but is not limited thereto. In addition, the pharmaceutical composition of the present disclosure may further include a diluent or an excipient, such as fillers, weighting agents, bonding agents, wetting agents, disintegrating agents and surfactants, and other pharmaceutically acceptable additives.

The pharmaceutical composition according to the present disclosure may be administered in a pharmaceutically effective amount. The term "pharmaceutically effective amount" refers to an amount sufficient to prevent or treat a disease at a reasonable benefit/risk ratio applicable to a medical treatment. The effective amount of the pharmaceutical composition of the present disclosure may be determined by a person with ordinary skill in the art according to various factors such as a formulation method, a patient's condition and weight, the patient's gender, age and degree of disease, a drug form, an administration route and period, an excretion rate, reaction sensitivity, etc. The effective amount may vary depending on a route of treatment, a use of excipients and a possibility of being used with other drugs, as recognized by a person with ordinary skill in the art.

The pharmaceutical composition of the present disclosure may be administered to mammals such as mice, livestock, humans, etc. through various routes. Specifically, the pharmaceutical composition of the present disclosure can be administered orally or parenterally (for example, applied or injected intravenously, subcutaneously or intraperitoneally). A solid preparation for oral administration may include powder, granule, tablet, capsule, soft capsule, pill, etc. A liquid preparation for oral administration may include suspension, liquid for internal use, emulsion, syrup, aerosol, etc., but may also include various excipients, for example, wetting agent, sweetener, flavoring agent and preservative in addition to generally used simple diluents such as water and liquid paraffin. A preparation for parenteral administration may be used by being formulated into a dosage form of external preparation and sterilized injectable preparation such as sterilized aqueous solution, liquid, water-insoluble excipient, suspension, emulsion, eye drop, eye ointment, syrup, suppository and aerosol according to respective conventional methods. Specifically, the pharmaceutical composition may be used in the form of cream, gel, patch, spraying agent, ointment, plaster, lotion, liniment, eye ointment, eye drop, paste, or cataplasm, but is not limited thereto. A preparation for topical administration may be an anhydrous or aqueous form depending on a clinical prescription. As the water insoluble excipient and the suspension, propylene glycol, polyethylene glycol, vegetable oil such as olive oil, and injectable ester like ethyl oleate, etc. may be used. Base materials of the suppository may include witepsol, macrogol, tween 61, cacao butter, laurinum and glycerogelatin.

### MODE FOR CARRYING OUT THE INVENTION

Hereinafter, the present disclosure will be explained in more detail with reference to Examples. However, the following Examples are exemplified only to explain the present disclosure but are not intended to limit the scope of the present disclosure.

### Example 1. Preparation of decellularized tissue-derived extracellular matrix (DT)

The porcine skin tissue was cut into pieces of 3×3×3 mm³ and then washed with distilled water for 12 to 16 hours. Thereafter, decellularization was performed using the following solutions sequentially: 1% Triton X-100 (Wako, Osaka, Japan) supplemented with 0.1% ammonium hydroxide (Sigma, St. Louis, MO, USA) for 3 days; distilled water (DW) for 2 days; 1% penicillin-streptomycin (Thermo Fisher Scientific, Waltham, MA, USA) for 2 hours; and distilled water (DW) for 2 hours.

The solutions used for decellularization and washing were replaced with fresh solutions twice a day. All processes were performed using a 4°C shaker (180 rpm). The decellularized tissue (hereinafter, referred to as "DT") was lyophilized and cold-stored until used.

### Example 2. Preparation of decellularized tissue-derived extracellular matrix functionalized with phenol derivative (DT-CA)

The decellularized tissue was made into a solution with stirring for 48 hours using a pepsin solution (4 mg/ml in 0.02 M hydrochloric acid, Sigma) (10 mg/ml). The decellularized tissue made into a solution was diluted to a concentration of 1 mg/ml using 0.01 M HCl, and the pH of the solution was adjusted to 4.5 using 1 M NaOH (Sigma). Thereafter, 1-(3-dimethylaminopropyl)-3-ethylcarbodiimide hydrochloride (EDC, Thermo Fisher Scientific) and N-hydroxysuccinimide (NHS, Sigma) were added to the solution to start the reaction. The molecular weight of DT was assumed to be 200 kg/mol, and the ratio of DT:EDC:NHS was adjusted to 1:5:5 (molar ratio). After the solution was allowed to react for 30 minutes, dopamine hydrochloride (Sigma) or 5-hydroxydopamine hydrochloride (Sigma) was added to DT at a ratio of 1: 5 (dopamine: DT, molar ratio) and then, the reaction was carried out overnight at pH 4.5. Dopamine (or hydroxydopamine), which did not participate in the reaction, was removed sequentially in acidic PBS (pH 3.5) and acidic distilled water (pH 3.5) by using a Cellu Sep with a 6 to 8 KDa cutoff (Membrane Filtration Products Inc., Seguin, TX, USA). The final product (hereinafter, referred to as "DT-CA") was lyophilized and stored at -20°C until used.

### Example 3. Confirmation of synthesis of decellularized tissue-derived extracellular matrix (DT) and decellularized tissue-derived extracellular matrix functionalized with phenol derivative (DT-CA)

A test was performed to analyze DT and DT-CA synthesized in Examples 1 and 2. Briefly, for histological analysis, each tissue before and after decellularization was fixed with 4% paraformaldehyde (Sigma) and then fixed in paraffin to prepare 6-µm tissue slices, followed by H&E staining (hematoxylin and eosin). Collagen type I (Col I) in the tissue was immunostained with anti-Col I primary antibodies (1:200 dilution, Calbiochem, San Diego, CA, USA) and Alexa 594-conjugated secondary antibodies (Invitrogen, Carlsbad, CA, USA), and the cell nuclei were stained with 4',6-diamidino-2-phenylindole (DAPI) (Vector Laboratories, Burlingame, CA, USA). The stained tissue was photographed with a confocal laser scanning microscope (LSM 880, Carl Zeiss).

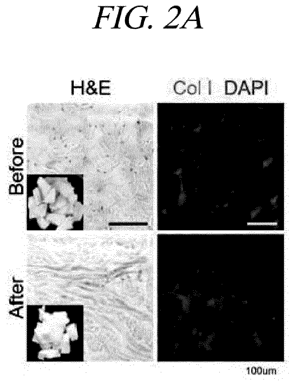

As a result, the decellularized porcine skin tissue appeared white, and it was confirmed by histological analysis that most of the cells were removed after decellularization, but the extracellular matrix including collagen (Col I) was well maintained **(****FIG. 2A****).**

Further, a test was performed to analyze the amount of DNA and glycosaminoglycan (GAG) before and after decellularization. Briefly, the amount of DNA remaining in the lyophilized tissue was quantified with a DNA detection kit (Bioneer, Daejeon, Korea) and the amount of glycosaminoglycan (GAG) was quantified with 1,9-dimethyl methylene blue (Sigma).

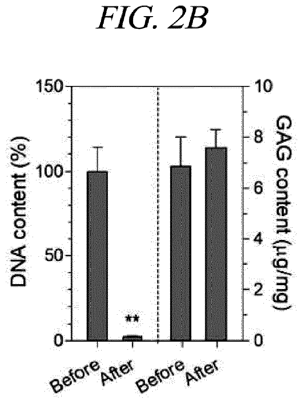

As a result, it was confirmed that about 98% of the cells were removed from the decellularized tissue through DNA quantification while the amount of glycosaminoglycan (GAG) was maintained at a level similar to that before decellularization **(****FIG. 2B****).**

Furthermore, a test was performed to determine whether the catechol group was well bound to the decellularized tissue. Briefly, 1 mg/ml of the decellularized tissue-derived extracellular matrix (DT) and the decellularized tissue-derived extracellular matrix functionalized with a catechol group (DT-CA) were dissolved in deuterium oxide (Sigma) and then, hydrogen nuclear magnetic resonance (¹H-NMR, 300 MHz, Bruker, Billerica, MA, USA) was used to determine whether the catechol group was well bound to the decellularized tissue.

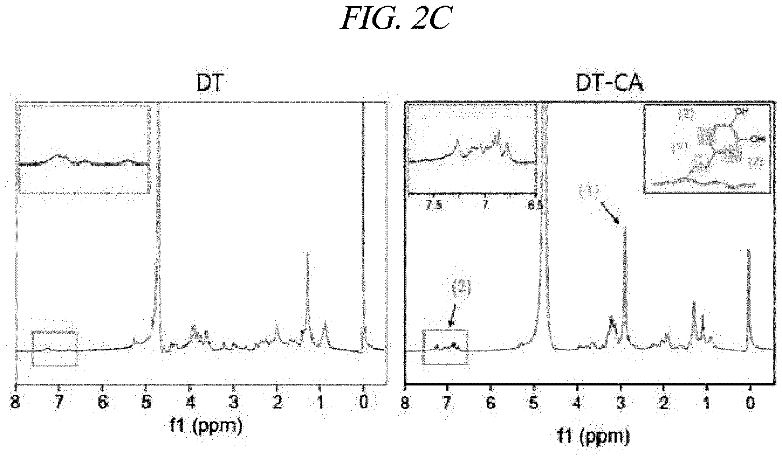

As a result, the NMR spectrum of the decellularized tissue-derived extracellular matrix functionalized with a catechol group (DT-CA) showed the presence of an ethyl proton (1) and a phenyl proton (2) between the catechol group and the DT backbone, which confirms that the decellularized tissue was well functionalized with a catechol group **(****FIG. 2C****).**

### Example 4. Preparation and characterization of decellularized tissue-derived extracellular matrix-based hydrogel functionalized with phenol derivative (DT-CA hydrogel)

A hydrogel was prepared by temperature crosslinking or oxidative crosslinking. Briefly, for preparation of a hydrogel (hereinafter, referred to as "tDT-CA hydrogel") by self-assembly, which is a temperature crosslinking method, a DT (20 mg/ml in 0.02 M HCl) solution and a DT-CA (20 mg/ml in distilled water) solution were prepared at a final concentration of 10 mg/ml by using 10× PBS (10% final concentration, Sigma) and distilled water. Immediately after the solution was adjusted to a final pH of 7.4 with 0.5 N NaOH, it was gelated at 37°C for 30 minutes.

For production of a hydrogel (hereinafter, referred to as "oDT-CA hydrogel") by oxidative crosslinking, DT-CA was dissolved in PBS and then, an oxidation reaction was performed by adding an oxidizer (sodium periodate, NaIO₄, Sigma). The final concentration of DT-CA in the DT-CA solution was 10 mg/ml, and the oxidizer was added in a ratio of 2 : 1 (NaIO₄ : dopamine, molar ratio) with respect to dopamine.

As a control group, a decellularized tissue-derived extracellular matrix-based hydrogel (hereinafter, referred to as "DT hydrogel"), which was not functionalized with a catechol group, was used.

An internal structure of the hydrogel was observed using a field-emission scanning electron microscope (FE-SEM) (7001F, JEOL, Tokyo, Japan). For preparation of samples, the DT hydrogel, the tDT-CA hydrogel and the oDT-CA hydrogel were sequentially transferred to each of 50%, 60%, 70%, 80%, 90% and 100% ethanol and t-butyl alcohol (Sigma) for 30 minutes, and then lyophilized.

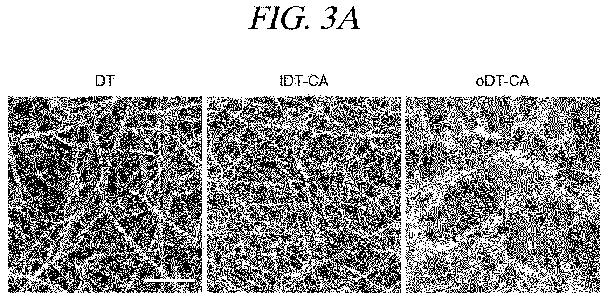

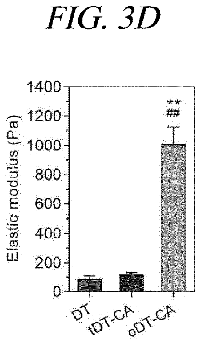

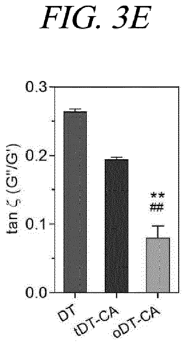

It was confirmed that the hydrogel may have various internal structures depending on the crosslinking method. In particular, it was observed that the tDT-CA hydrogel which was prepared by temperature crosslinking had an internal structure in the form of nanofibers similar to that of the DT hydrogel which was not functionalized with a catechol group, whereas the oDT-CA hydrogel which was prepared by oxidative crosslinking had a porous internal structure **(****FIG. 3A****).**

Physical properties were analyzed using a rotary rheometer (MCR 102, Anton Paar, Ashland, VA, USA). A storage modulus (G') and a loss modulus (G") of the hydrogel were measured within the range of 0.1 Hz to 10 Hz in a frequency sweep mode (1% strain condition). The average storage modulus at 1 Hz was taken as an elastic modulus. As for a gelation rate, G' and G" were measured in a time sweep mode (10% strain, 1 Hz frequency) and the time for which G' and G" crossed over was taken as gelation time.

In particular, the gelation rate of the hydrogel through self-assembly was measured by placing a pre-gel solution on a base plate set at 4°C and then increasing the temperature to 37°C. In a relaxation test, a relaxation stress over time was measured (10% shear strain).

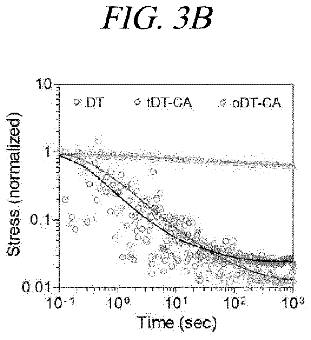

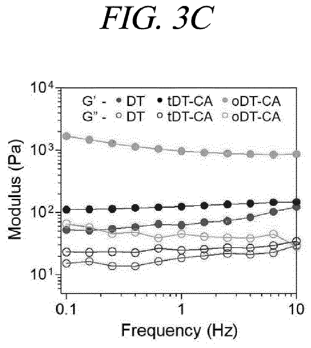

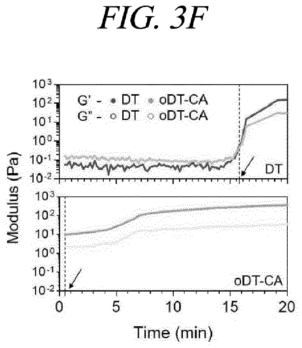

As a result, the relaxation test confirmed that the oDT-CA hydrogel composed of covalent bonds/ irreversible bonds was physically more stable than the DT hydrogel or tDT-CA hydrogel composed of ionic bonds/reversible bonds **(****FIG. 3B****).** Also, it was confirmed that the oDT-CA hydrogel (1055.1 ± 82.3 Pa) increased in physical properties (modulus) by about 10 times compared with the DT hydrogel or tDT-CA hydrogel (119.2 ± 14.2 Pa for the tDT-CA hydrogel) **(****FIG. 3C** to **FIG. 3E****).** Further, it was confirmed from rheometer analysis that the oDT-CA hydrogel was gelated within several tens of seconds, which confirms that the crosslinking reaction rate of the oDT-CA hydrogel is significantly higher than that of the DT hydrogel that was gelated within 15 to 20 minutes **(****FIG. 3F****).**

From the above results, it was confirmed that the decellularized tissue-derived extracellular matrix-based hydrogel functionalized with a catechol group (DT-CA hydrogel) increased in physical properties and had a significantly high crosslinking reaction rate compared with the decellularized tissue-derived extracellular matrix-based hydrogel (DT hydrogel) that was not functionalized with a catechol group.

### Example 5. Analysis of structural stability and tissue adhesion of DT-CA hydrogel

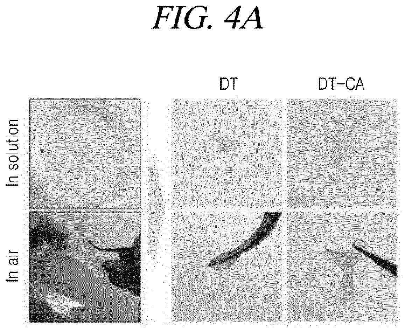

A test was performed to check the structural stability of the DT-CA hydrogel. Briefly, each hydrogel having a "Y" shape was prepared, and whether or not the "Y" shape was maintained was checked inside the aqueous solution (in solution) and outside the aqueous solution (in air). As a result, it was confirmed that the "Y" shape of the DT hydrogel was maintained only inside the aqueous solution after gelation, whereas the "Y" shape of the DT-CA hydrogel was well maintained not only inside the aqueous solution but also outside the aqueous solution **(****FIG. 4A****).**

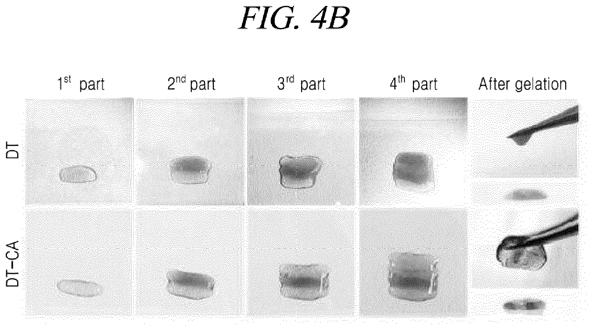

Also, a test was performed to examine the applicability of additive manufacturing. Briefly, pre-gel solutions containing green or red dyes were drawn sequentially and then gelated. As a result, the DT hydrogel which was gelated slowly and had weak physical properties had layers, which collapsed during the drawing process, and did not maintain its shape even after gelation, whereas the DT-CA hydrogel which was gelated fast and had enhanced physical properties maintained the original shape as being drawn **(****FIG. 4B****).**

Further, a test was performed to check swelling/ degradation patterns of the DT-CA hydrogel. Briefly, in order to check swelling and degradation, each gelated hydrogel was immersed in a PBS or collagenase solution (0.1 mg/ml, Sigma) and then cultured at 37°C while changes in weight were measured. The degree of swelling/degradation of the hydrogel was calculated using the following equation: Swelling/Degradation Ratio = (Wt - Wi)/Wi × 100. In the above equation, Wt represents the weight of the hydrogel at a specific time, and Wi represents the weight of the hydrogel before the start of culture.

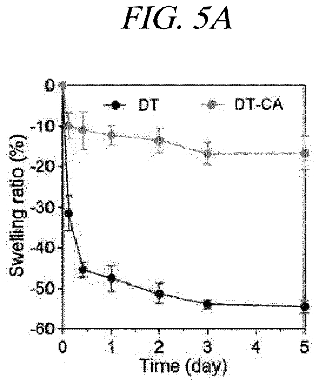

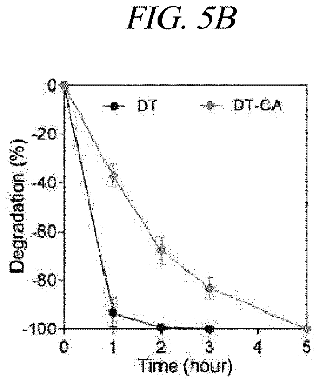

As a result, it was confirmed that the DT hydrogel was shown to rapidly deswell, whereas the DT-CA hydrogel showed a significant decrease in degree of deswelling due to enhanced physical properties **(****FIG.** 5A). Further, as a result of checking degradation patterns using a collagenase, more than 90% of the DT hydrogel was degraded within 1 hour and almost all of the DT hydrogel was degraded within 2 hours, whereas only about 80% of the DT-CA hydrogel was degraded within 3 hours and the DT-CA hydrogel showed a significant decrease in degradation rate compared with the DT hydrogel **(****FIG. 5B****).**

Also, a test was performed to check a tissue adhesive property of the DT-CA hydrogel. Briefly, the tissue adhesive property was measured by placing each pre-gel solution in the mouse skin (fixed to the plate with a strong adhesive), sufficiently gelating the pre-gel solution by each method, and then measuring adhesion in a tack-separation mode (at a rate of 10 µm/s).

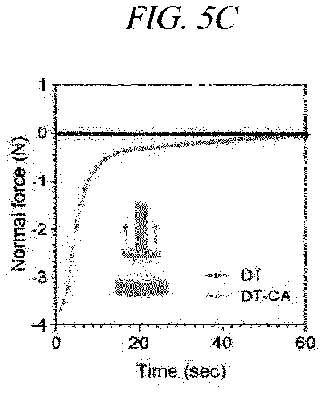

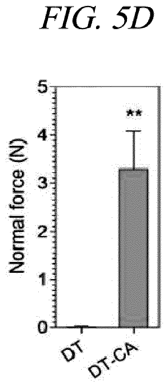

As a result, the DT hydrogel has a normal force close to 0, whereas the DT-CA hydrogel has a normal force of about 3 N. Thus, it was confirmed that the DT-CA hydrogel has a tissue adhesive property which does not appear in the DT hydrogel **(****FIG. 5C** and **FIG. 5D****).**

From the above results, it was confirmed that the decellularized tissue-derived extracellular matrix-based hydrogel functionalized with a catechol group (DT-CA hydrogel) was improved in structural stability compared with the decellularized tissue-derived extracellular matrix-based hydrogel (DT hydrogel) which was not functionalized with a catechol group and could form a stable three-dimensional structure without an additional polymer scaffold. Further, it was confirmed that the DT-CA hydrogel had a tissue adhesive property which is very important for stable engraftment and maintenance after implantation of a biomaterial. Thus, it can be suitable as a biomaterial for tissue regeneration.

### Example 6. Analysis of structural stability and biocompatibility of DT-CA hydrogel upon cell encapsulation

A test was performed to check the structural stability and biocompatibility of the DT-CA hydrogel during cell encapsulation. Briefly, human adipose-derived stem cells (hADSCs) were purchased from ATCC (American Type Culture Collection, Rockville, MD, USA) and cultured using a mesenchymal stem cell (MSC) growth medium (ATCC) at 37°C in a 5% CO₂ incubator. The hADSCs were mixed with each pre-gel solution at a concentration of 1 × 10⁷ cells/ml and gelated by self-assembly or oxidation. In order to check whether the hydrogel shrinks during cell encapsulation, a hydrogel of the same size (5 mm) was prepared, and changes in diameter over time (0 h, 4 h, 9 h, 1 d, 14 d, 21 d) were measured. The shrinkage of the hydrogel was calculated using the following equation: shrinking ratio = (Dt - Di)/Di × 100. In the above equation, Dt represents the diameter of the hydrogel at a specific time, and Di represents the diameter of the hydrogel before culture.

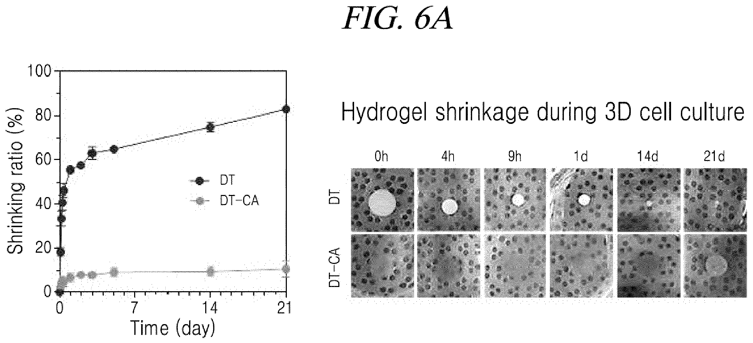

As a result, the DT hydrogel with encapsulated stem cells was shown to rapidly shrink over time. In particular, it was confirmed that the hydrogel shrank to almost 20% or less on day 21, the end of the test **(****FIG. 6A****).** Also, it was confirmed that the DT-CA hydrogel with encapsulated stem cells hardly shrank even on day 21, the end date of the test **(****FIG. 6A****).**

Also, the encapsulated cells were stained with a live/dead viability/cytotoxicity kit (Invitrogen) to measure the cell viability under a fluorescence microscope. As a control group, a collagen type I extracellular matrix-based hydrogel functionalized with a catechol group (hereinafter, referred to as "Col-CA hydrogel") using collagen type I (Col I), which is an extracellular matrix widely used in tissue engineering, was used.

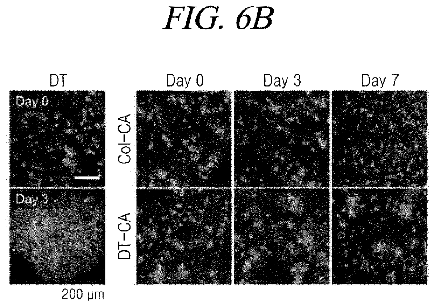

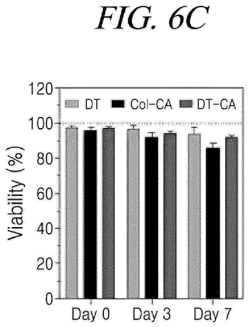

As a result, it was confirmed that the hADSC cells were well maintained when cells were encapsulated in the DT-CA hydrogel and the Col-CA hydrogel, which confirms that the DT-CA hydrogel has high biocompatibility and low cytotoxicity **(****FIG. 6B** and **FIG. 6C****).**

From the above results, it was confirmed that the decellularized tissue-derived extracellular matrix-based hydrogel functionalized with a catechol group (DT-CA hydrogel) was improved in structural stability due to the enhanced physical properties, and stem cells could be cultured in the hydrogel due to its high biocompatibility and low cytotoxicity.

### Example 7. Analysis of efficacy of DT-CA hydrogel for enhancing stem cell differentiation

A test was performed to determine whether the DT-CA hydrogel has the effect of enhancing or improving the stem cell differentiation capability. Briefly, osteodifferentiation was induced in hADSC cells in the hydrogel by using the following culture medium for inducing osteodifferentiation: DMEM medium (Dulbecco's Modified Eagle's Medium, Gibco, Gaithersburg, MD, USA) containing 100 nM dexamethasone (Sigma); 50 µg/ml L-ascorbic acid (Sigma); 10 mM β-glycerophosphate (Sigma); 10% (v/v) FBS (fetal bovine serum, Gibco); 3.7 g/L sodium bicarbonate (Sigma); and 1% (v/v) penicillin/streptomycin (Gibco). After differentiation was induced for 3 weeks, cell differentiation patterns were checked through immunostaining and real-time polymerase chain reaction (real time-PCR).

The real time-PCR confirmed the expression levels of OPN (human osteopontin, Hs00959010_m1) and OCN (human osteocalcin, Hs01587814_g1), which are osteocyte-specific markers, and GAPDH (human glyceraldehyde-3-phosphate dehydrogenase, Hs02758991_g1) was used as a housekeeping gene.

For immunostaining analysis, the hydrogel was fixed with 4% paraformaldehyde (Sigma) for 2 days and then staining was performed with primary antibodies, such as anti-Osteopontin (1:100, Santa Cruz Biotechnology Inc., Santa Cruz, CA, USA), anti-Fibronectin (1:100, Abcam, Cambridge, UK), and secondary antibodies, such as Alexa-Fluor 488-conjugated secondary antibody and Alexa-Fluor 594-conjugated secondary antibody (Invitrogen), and the cell nuclei were stained with DAPI (Vector Laboratories). The stained cells were photographed with a confocal laser scanning microscope (LSM 880, Carl Zeiss).

For Alizarin red staining, the differentiated cells were stained with an Alizarin red solution (Sigma) for 5 minutes, and the excess solution was sufficiently removed with PBS, followed by photographing with a microscope.

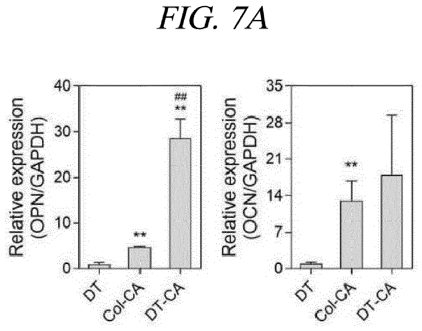

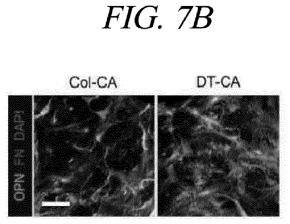

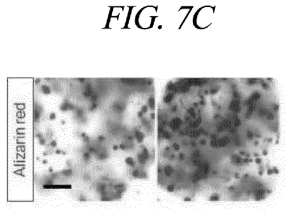

As a result, the stem cells in the DT-CA hydrogel showed a significantly higher expression level of OPN mRNA than the stem cells in the DT hydrogel or Col-CA hydrogel, and showed a much higher expression level of OPN mRNA than the stem cells in the DT hydrogel, which confirms that the DT-CA hydrogel has the effect of significantly enhancing osteodifferentiation **(****FIG. 7A****).** Also, it was further confirmed through immunostaining for OPN and Alizarin red staining that osteodifferentiation was more significantly enhanced in the stem cells in the DT-CA hydrogel than in the stem cells in the Col-CA hydrogel **(****FIG. 7B** and **FIG. 7C****).**

From the above results, it was confirmed that the decellularized tissue-derived extracellular matrix-based hydrogel functionalized with a catechol group (DT-CA hydrogel) has the effect of significantly enhancing or promoting stem cell differentiation compared with the decellularized tissue-derived extracellular matrix-based hydrogel (DT hydrogel) that was not functionalized with a catechol group.

### Example 8. Analysis of efficacy of DT-CA hydrogel for enhancing tissue regeneration

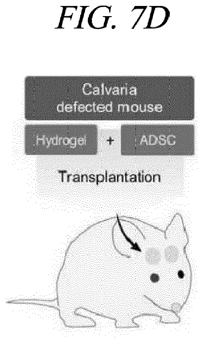

A test was performed to analyze the efficacy of a hydrogel for enhancing bone regeneration in a calvaria defect mouse model. Briefly, all animal tests were approved by the Institutional Animal Care and Use Committee of Yonsei University (IACUC-201801-692-02). The calvaria defect animal model was prepared by removing 4 mm in size from the mouse calvaria. After 5 × 10⁵ cells were encapsulated in each hydrogel (50 µL), the hydrogel was implanted to the defect site **(****FIG. 7D****).** In particular, the DT-CA hydrogel was implanted in the defect site immediately after pre-gel was mixed with an oxidizer, and then gelated for 10 minutes to maintain a tissue adhesive property. At 8 weeks after implantation, the calvaria was collected, fixed in 4% paraformaldehyde (Sigma) and treated with a decalcifying solution (Sigma) at room temperature for 4 hours. Thereafter, the tissue was fixed in paraffin and cut to a thickness of 6 µm to prepare a sample.

Collagen formation in the tissue was confirmed by Goldner's Trichrome staining. Further, staining was performed with primary antibodies, such as anti-OPN (1:100, Santa Cruz Biotechnology Inc.) anti-Col I (1:200, Calbiochem), and secondary antibodies, such as Alexa-Fluor 488-conjugated secondary antibody and Alexa-Fluor 594-conjugated secondary antibody (Invitrogen), and the cell nuclei were stained with DAPI (Vector Laboratories). The stained tissue was photographed with a confocal laser scanning microscope (LSM 880, Carl Zeiss). As for the degree of bone regeneration in the bone defect site, the expression levels of OPN and Col I were quantified with ImageJ software (National Institutes of Health, Bethesda, MD, USA) based on the photographed images.

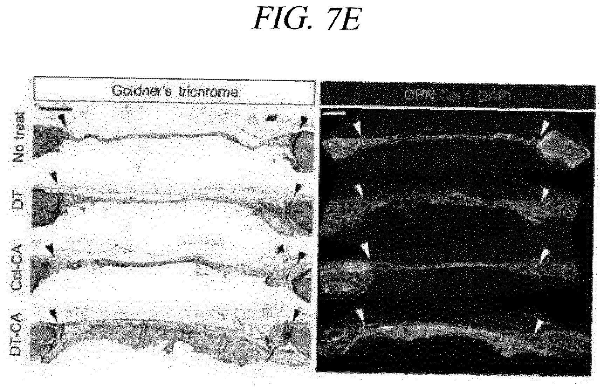

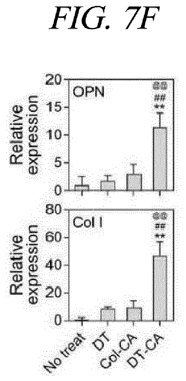

As a result, it was confirmed that the expression levels of OPN and Col I were more significantly increased in the group implanted with the DT-CA hydrogel together with the stem cells than in the group implanted with the DT hydrogel or Col-CA hydrogel together with the stem cells, which confirms that the DT-CA hydrogel has a significant effect on bone regeneration **(****FIG. 7E** and **FIG. 7F****).**

### Example 9. Preparation and characterization of decellularized bone tissue-derived extracellular matrix-based hydrogel functionalized with phenol derivative (Bone-CA hydrogel)

The porcine bone tissue was cultured in a 6% hydrogen peroxide solution (Sigma) for 3 days and then lyophilized and cut into pieces of 4×4×4 mm³ or less. Thereafter, decellularization was performed using the following solutions: 0.5 N hydrogen chloride (Sigma) for 3 days; 70% ethanol overnight; 0.6 N citric acid (Sigma) for 3 days; distilled water overnight; and phosphate-buffered saline (PBS, Sigma) supplemented with 1% penicillin-streptomycin (Sigma) overnight.

Further, a decellularized tissue-derived extracellular matrix functionalized with a phenol derivative (catechol group) (hereinafter, referred to as "Bone-CA") was prepared from the decellularized bone tissue by the method as in Example 2, and a decellularized bone tissue-derived extracellular matrix-based hydrogel functionalized with a phenol derivative (catechol group) (hereinafter, referred to as "Bone-CA hydrogel") was prepared by oxidative crosslinking as in Example 4. As a control group, a bone tissue-derived extracellular matrix-based hydrogel (hereinafter, referred to as "Bone hydrogel") prepared by temperature crosslinking was used.

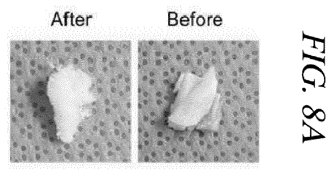

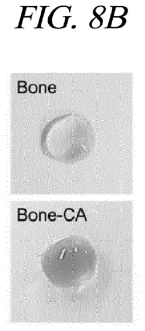

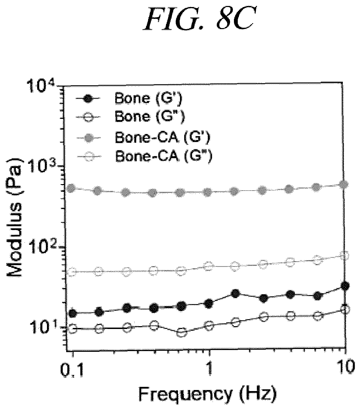

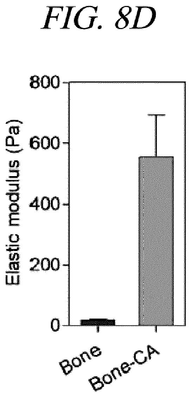

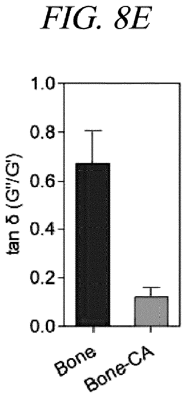

The decellularized porcine bone tissue appeared white **(****FIG. 8A****),** and it was confirmed that the hydrogel was well formed by oxidative crosslinking **(****FIG. 8B****).** Further, it was confirmed that the Bone-CA hydrogel prepared by oxidative crosslinking increased in physical properties by about 10 times compared with the Bone hydrogel prepared by temperature crosslinking, and also greatly increased in elasticity **(****FIG. 8C** to **FIG. 8E****).**

From the above results, it was confirmed that the hydrogel functionalized with a catechol group and prepared by oxidative crosslinking showed improved physical properties even in decellularized bone tissue.

### Example 10. Analysis of drug delivery ability of DT-CA hydrogel

A test was performed to check drug encapsulation and release patterns of the DT-CA hydrogel. Briefly, human VEGF (vascular endothelial growth factor, R&D systems, MN, USA) was encapsulated in each hydrogel at a concentration of 1 µg/m!, and cultured at 37°C in PBS. The amount of drug released was measured by replacing the culture medium at a specific time. The amount of VEGF in the solution was quantified with a human VEGF ELISA kit (R&D Systems).

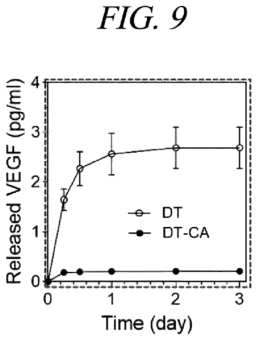

As a result, a very fast initial release of the drug was observed in the DT hydrogel, whereas only a very small amount of the drug was released from the DT-CA hydrogel **(****FIG. 9****).**

From the above results, it was confirmed that the decellularized tissue-derived extracellular matrix functionalized with a catechol group (DT-CA hydrogel) can more stably maintain the drug encapsulated in the hydrogel and can continuously release the drug for a longer time than the decellularized tissue-derived extracellular matrix-based hydrogel (DT hydrogel) which was not functionalized with a catechol group, which confirms that the DT-CA hydrogel can be used for drug delivery.

### Example 11. Preparation and characterization of DT-CA hydrogel patch, and analysis of drug delivery ability

A patch was manufactured using the DT-CA hydrogel and a test was performed to analyze its characteristics. Briefly, 200 µL of a DT-CA solution (10 mg/ml) was injected into a specific mold and then lyophilized. For crosslinking of the DT-CA hydrogel patch, the DT-CA hydrogel patch was placed on the desired site and then, an oxidizer (4.5 mg/ml NaIO₄) was sprayed thereto.

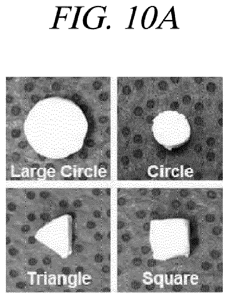

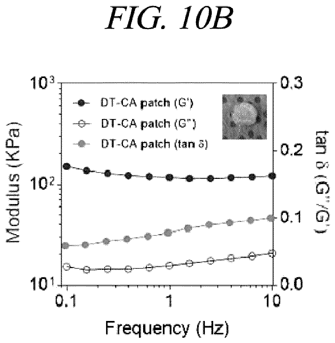

The DT-CA hydrogel patch could be prepared in various shapes **(****FIG. 10A****),** and exhibited higher physical properties by more than 100 times than the oDT-CA hydrogel prepared by oxidative crosslinking and enhanced in physical properties (at 1 Hz, oDT-CA = 1.01 ± 0.1 KPa, DT-CA hydrogel patch = 119.7 ± 19.3 KPa). Also, the DT-CA hydrogel patch had an elasticity value, which is a median between the values for the tDT-CA hydrogel and the oDT-CA hydrogel **(****FIG. 10B****).**

Also, a test was performed to check a drug release pattern of the DT-CA hydrogel patch with the encapsulated drug. Briefly, human EGF (epidermal growth factor, Peprotech, Rocky Hill, NJ, USA) was encapsulated at a concentration of 5 µg/ml, and cultured at 37°C in a PBS or collagenase A solution (2 mg/ml, Sigma). The amount of drug released was measured by replacing the culture medium at each given time. The amount of EGF in the solution was quantified with a human EGF ELISA kit (R&D Systems).

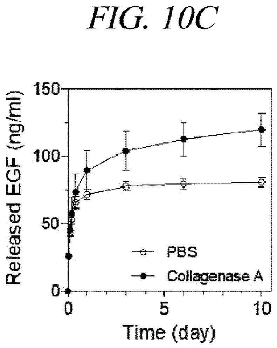

As a result, a relatively small amount of growth factor was released from the PBS solution in which the patch was not degraded, whereas the encapsulated growth factor was slowly released from the DT-CA hydrogel patch while the DT-CA hydrogel patch was biodegraded in the collagenase A solution similar to the in vivo environment **(****FIG. 10C****).**

From the above results, it was confirmed that the DT-CA hydrogel patch has enhanced physical properties and thus can effectively encapsulate a drug and can continuously release the drug for a long time by in vivo biodegradation of the hydrogel, which confirms that the DT-CA hydrogel patch can be used for drug delivery.

### Example 12. Analysis of efficacy of DT-CA hydrogel patch for treating wound or promoting tissue regeneration

A test was performed to analyze the efficacy of the DT-CA hydrogel patch for wound treatment in a wound animal model. Briefly, a biopsy punch was used to induce a circular wound having a diameter of 8 mm in the dorsal skin tissue of a mouse (SKH1-hr, 6 weeks old, male, OrientBio, Seongnam, Korea). Then, the wound of the mouse was treated with a solution-based DT-CA hydrogel (Bulk) or DT-CA hydrogel patch (Patch), and also treated with a DT-CA hydrogel (Bulk+EGF) or DT-CA hydrogel patch (Patch+EGF) with encapsulated EGF (1 µg/mouse) to verify the efficacy for EGF delivery. The condition and treatment aspect of the mouse were checked for 12 days after preparation of the wound animal model and material implantation, and the blood flow at the wound site of the mouse was measured with a laser Doppler imaging device (Moor Instruments, Devon, UK) on day 12 after material implantation. In the damaged skin tissue, early microvascularization occurred vigorously, but as the tissue organelles were formed in the dermal layer over time, the number of microvessels was relatively decreased. Thus, the efficacy for wound treatment was inferred by analyzing the blood flow in the skin surface.

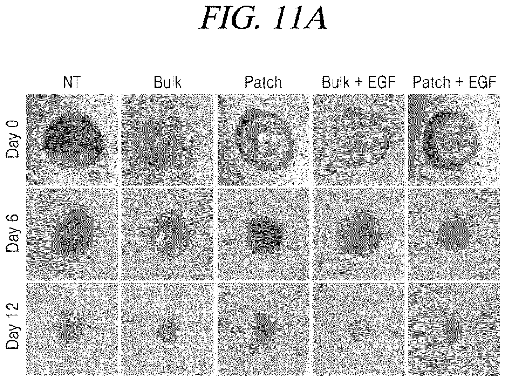

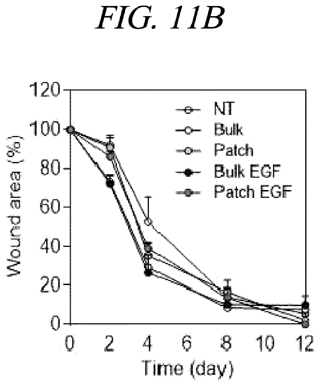

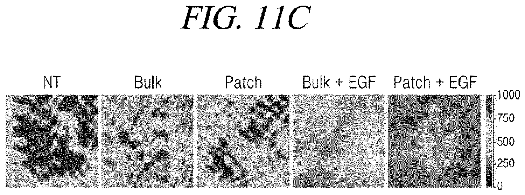

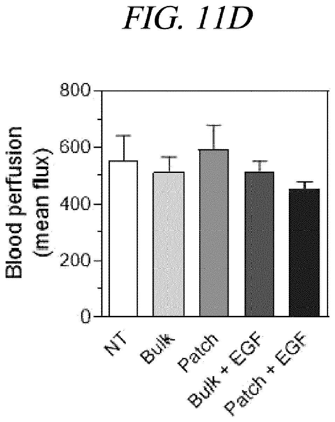

As a result, the size of the wound was most effectively reduced in the group treated with the DT-CA hydrogel patch or the DT-CA hydrogel patch with the encapsulated drug (Patch+EGF) **(****FIG. 11A** and **FIG. 11B****).** Further, it was confirmed from laser Doppler imaging analysis that the blood flow was significantly decreased in the group treated with the DT-CA hydrogel patch with the encapsulated drug (Patch+EGF) **(****FIG. 11C** and **FIG. 11D****).** Thus, it was confirmed that the DT-CA hydrogel patch with the encapsulated drug has the most remarkable effect on wound treatment.

Also, a histological analysis was performed to the wound site of the wound animal model. Briefly, the skin tissue was collected on day 12 after material implantation, fixed in 10% formalin (Sigma) and then fixed in paraffin to prepare 6-µm tissue slices, followed by each of H&E staining and Masson's trichrome staining (Sigma) for staining ECM and collagen in the tissue. Further, immunostaining was performed with primary antibodies, such as anti-Keratin 14 (Abcam) and anti-Involucrin (Abcam), and secondary antibodies, such as Alexa-Fluor 488-conjugated secondary antibody and Alexa-Fluor 594-conjugated secondary antibody (Invitrogen), and the cell nuclei were stained with DAPI (Vector Laboratories) to check regeneration of the basal layer and the stratum corneum of the skin tissue. The stained tissue was photographed with a confocal laser scanning microscope (LSM 880, Carl Zeiss). In order to check skin tissue regeneration, the wound area, the epidermal thickness, the amount of collagen and the number of hair follicles in the wound area were measured based on the photographed images. ImageJ software (National Institutes of Health, Bethesda, MD, USA) was used to analyze the images.

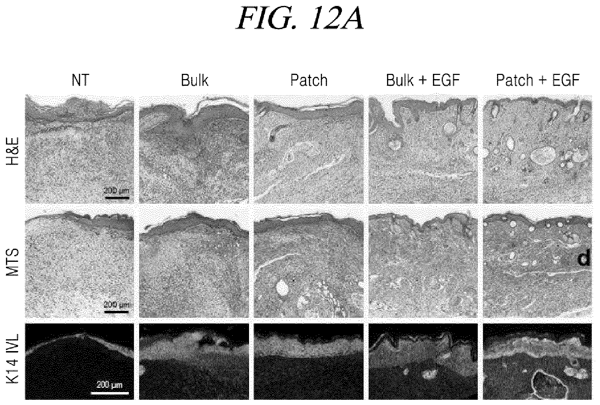

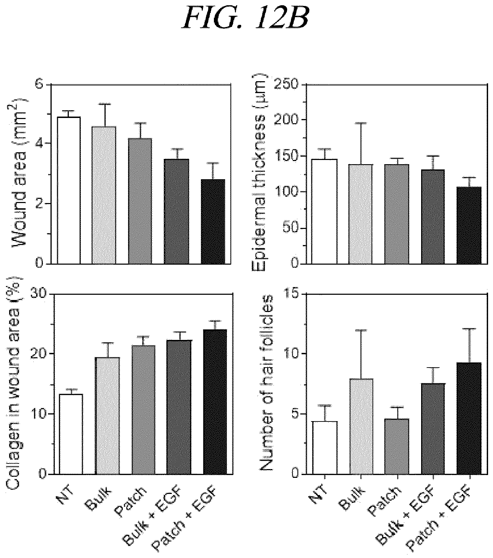

As a result, it was confirmed from H&E staining and Masson's trichrome staining that in the DT-CA hydrogel patch group with the encapsulated drug (Patch+EGF), the abnormal skin tissue had the smallest area and the epidermal layer had the smallest thickness (the tissue has a greater thickness when damaged or having inflammation) with the greatest collagen accumulation area and the largest number of newly formed hair follicles, which confirms that effective skin regeneration occurred **(****FIG. 12A** and **FIG. 12B****).**

From the above results, it was confirmed that the DT-CA hydrogel patch has improved physical properties and tissue adhesive property and thus can be stably attached to the wound site, and, thus, the DT-CA hydrogel patch can protect the wound site from external stimuli. Also, it was confirmed that the DT-CA hydrogel patch can be used for effective drug delivery through drug encapsulation and thus can be used for promoting tissue regeneration or for tissue implantation.

### Example 13. Preparation and characterization of decellularized tissue-derived extracellular matrix-based hydrogel functionalized with phenol derivative (pyrogallol group) (DT-PG hydrogel)

A decellularized tissue-derived extracellular matrix functionalized with a pyrogallol group (PG), which has stronger oxidativity, instead of a catechol group among phenol derivatives was prepared in the same manner as in the above Example. Further, a hydrogel (hereinafter, referred to as "DT-PG hydrogel") was prepared by oxidative crosslinking from the decellularized tissue-derived extracellular matrix functionalized with a pyrogallol group in the same manner as in the above Example.

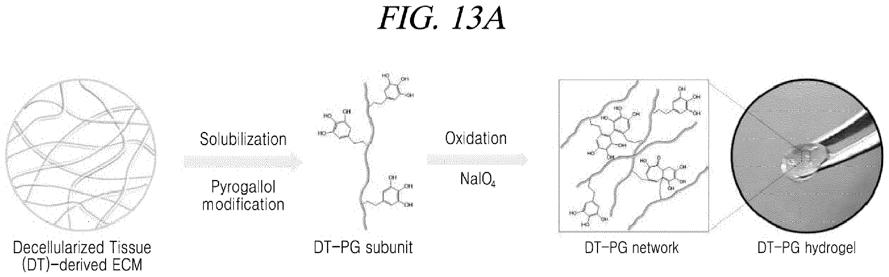

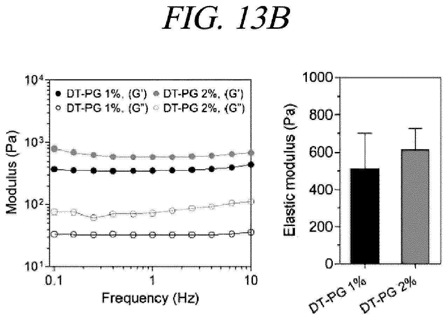

It was confirmed that the hydrogel was formed very fast when an oxidizer was added to the decellularized tissue-derived extracellular matrix functionalized with a pyrogallol group **(****FIG. 13A****).** Also, it was confirmed that the DT-PG hydrogel was further enhanced in physical properties than the DT hydrogel and its physical properties could be regulated depending on the concentration **(****FIG. 13B****).**

From the above results, it was confirmed that the decellularized tissue-derived extracellular matrix-based hydrogel functionalized with a phenol derivative, *i.e.*, a pyrogallol group instead of a catechol group, is enhanced in physical properties.

### Example 14. Analysis of efficacy of DT-PG hydrogel patch for treating wound or promoting tissue regeneration

The applicability of the DT-PG hydrogel patch was checked as a medical material for promoting skin regeneration in a wound animal model. Briefly, a biopsy punch was used to induce a circular wound having a diameter of 8 mm in the dorsal skin tissue of a mouse and then, the wound of the mouse was treated with a solution-based DT-PG hydrogel (Bulk) or DT-PG hydrogel patch (Patch). Also, EGF (1 µg/mouse) was delivered to promote skin tissue regeneration and thus to induce the DT-PG material to improve treatment efficacy through sustained drug release.

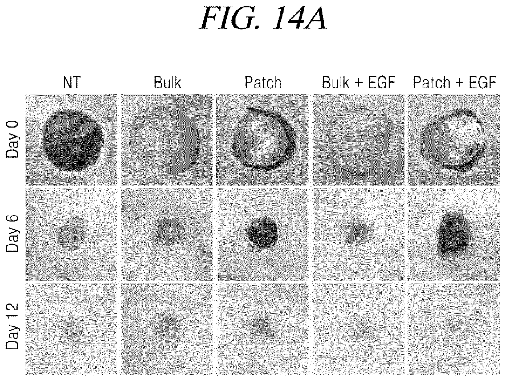

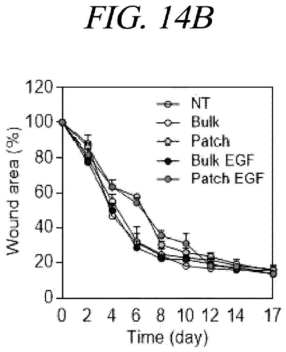

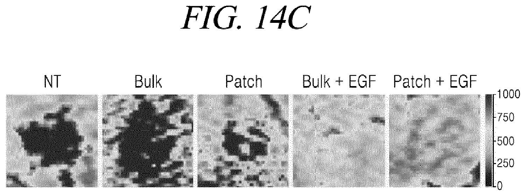

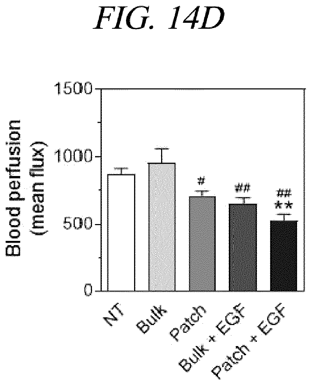

As a result, the groups did not show a significant difference in the size of the visually observed wound until day 17 after application of the patch **(****FIG. 14A** and **FIG. 14B****).** However, it was confirmed from laser Doppler imaging analysis on day 17 that the blood flow was significantly decreased in the group treated with the DT-PG hydrogel patch with the encapsulated drug (Patch+EGF) **(****FIG. 14C** and **FIG. 14D****).** In the damaged skin tissue, early microvascularization occurred vigorously, but as the tissue organelles were formed in the dermal layer over time, the number of microvessels was relatively decreased. Therefore, it can be inferred that the blood flow was greatly decreased in the group treated with the DT-PG hydrogel patch with the encapsulated drug (Patch+EGF), and, thus, the size of the wound was most significantly reduced.

From the above results, it was confirmed that the DT-PG hydrogel patch used in the present disclosure has improved physical properties and tissue adhesive property compared with the conventional hydrogel formulation and thus can be stably attached to the diseased site, and, thus, the DT-PG hydrogel patch can protect the wound site from external stimuli and can induce effective drug delivery.

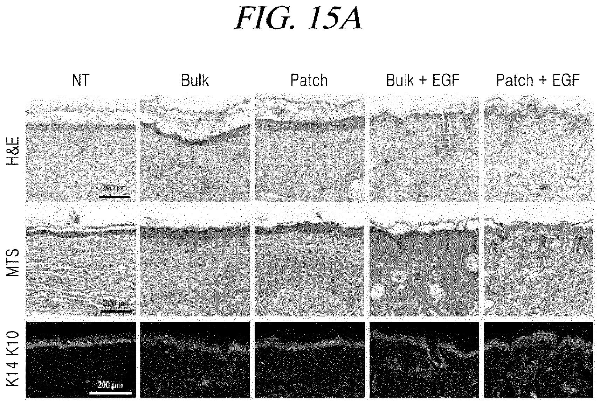

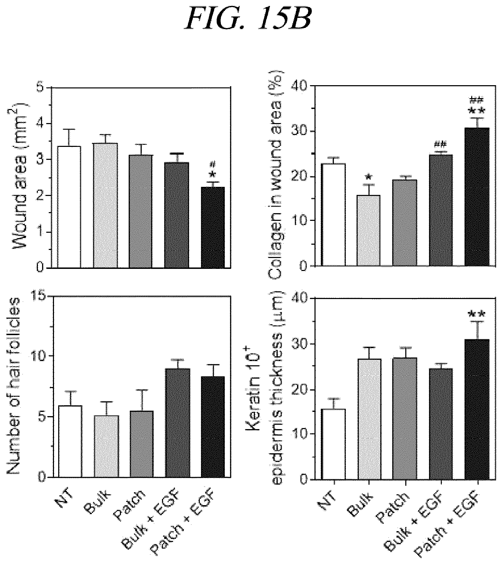

Further, in order to evaluate the efficacy of the DT-PG hydrogel and the patch-based drug delivery system for wound treatment, a tissue site where the wound was induced was collected on day 17 after induction of the model and application of the hydrogel, followed by histological analysis. As a result, it was confirmed from H&E staining and Masson's trichrome staining that in the DT-PG hydrogel patch group with the encapsulated drug (Patch+EGF), the abnormal skin tissue had the smallest area with the greatest collagen accumulation area and the increase in number of newly formed hair follicles. Also, it was confirmed from immunostaining for Keratin 10 and Keratin 14 that the Keratin 10-positive epidermal layer had the greatest thickness (the tissue has a smaller thickness when damaged or inflamed) **(****FIG. 15A** and **FIG. 15B****).**

Therefore, it was confirmed that the DT-PG patch system can induce effective tissue regeneration through stable drug delivery in vivo.

## Claims

1. A hydrogel including a decellularized tissue-derived extracellular matrix functionalized with a phenol derivative.

2. The hydrogel of Claim 1,
wherein the phenol derivative is a catechol group derived from a catechol-based compound selected from the group consisting of catechol, 4-tert-butylcatechol (TBC), urushiol, alizarin, dopamine, dopamine hydrochloride, 3,4-dihydroxyphenylalanine (DOPA), caffeic acid, norepinephrine, epinephrine, 3,4-dihydroxyphenylacetic acid (DOPAC), isoprenaline, isoproterenol and 3,4-dihydroxybenzoic acid; or
a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

3. The hydrogel of Claim 1,
wherein the tissue is selected from the group consisting of liver, heart, kidney, muscle, stomach, intestine, lung, bone, cartilage, blood vessel, bladder, skin, brain, fat, thyroid gland, salivary gland, esophagus, pancreas, spinal cord, ligament, tendon, tooth and uterus.

4. The hydrogel of Claim 1,
wherein 90% or more of cells are removed from the decellularized tissue-derived extracellular matrix.

5. The hydrogel of Claim 1,
wherein the hydrogel is prepared through oxidation of the functionalized phenol derivative.

6. The hydrogel of Claim 5,
wherein the oxidation is carried out by addition of an oxidizer or an enzyme.

7. The hydrogel of Claim 5,
wherein the oxidation is carried out by a body enzyme without addition of an oxidizer or an enzyme.

8. The hydrogel of Claim 1,
wherein the hydrogel has a porous structure.

9. The hydrogel of Claim 1,
wherein the hydrogel has an elastic modulus of from 100 Pa to 1500 Pa at 1 Hz.

10. The hydrogel of Claim 1,
wherein the hydrogel has a tissue adhesive property.

11. The hydrogel of Claim 1,
wherein the hydrogel is biodegradable.

12. A composition for cell culture including a hydrogel of any one of Claim 1 to Claim 11.

13. The composition of Claim 12,
wherein the cell culture is a three-dimensional culture.

14. A composition for promoting cell differentiation including a hydrogel of any one of Claim 1 to Claim 11.

15. The composition of Claim 14,
wherein the cell is a stem cell.

16. The composition of Claim 15,
wherein the stem cell is selected from the group consisting of embryonic stem cells, fetal stem cells, induced pluripotent stem cells and adult stem cells.

17. A composition for drug delivery including a hydrogel of any one of Claim 1 to Claim 11.

18. The composition of Claim 17,
wherein the drug is encapsulated or loaded in the hydrogel.

19. The composition of Claim 17,
wherein the drug is selected from the group consisting of an immune cell activator, an anticancer agent, a therapeutic antibody, an antibiotic, an antibacterial agent, an antiviral agent, an anti-inflammatory agent, a contrast medium, a protein drug, a growth factor, a cytokine, a peptide drug, a hair growth solution, an anesthetic and combinations thereof.

20. The composition of Claim 17,
wherein the composition is in the form of an adhesive patch or a film.

21. A composition for promoting tissue regeneration including a hydrogel of any one of Claim 1 to Claim 11.

22. A composition for tissue implantation including a hydrogel of any one of Claim 1 to Claim 11.

23. The composition of Claim 21 or Claim 22,
wherein the composition is in the form of an adhesive patch or a film.

24. A method of preparing a decellularized tissue-derived extracellular matrix-based hydrogel functionalized with a phenol derivative, comprising:
a process of functionalizing a decellularized tissue-derived extracellular matrix with a phenol derivative.

25. The method of Claim 24, further comprising:
a process of oxidizing the functionalized phenol derivative.
